# EUROPEAN PATENT SPECIFICATION

(11) **EP 1 212 278 B1**
(45) Date of publication and mention of the grant of the patent: **19.01.2005**
(21) Application number: 00959195.9
(22) Date of filing: 08.08.2000
(51) Int. Cl.: C07C 51/41, C07C 51/43, C07C 63/38, C07C 51/42

(54) **A PROCESS FOR SEPARATION OF CRUDE NAPHTHALENE DICARBOXYLIC ACID USING REVERSE OSMOSIS**
EIN VERFAHREN ZUR TRENNUNG VON ROHER NAPHTHALENEDICARBONSÄURE MITTELS UMKEHROSMOSE
SEPARATION D'ACIDE NAPHTALENE DICARBOXYLIQUE BRUT AU MOYEN D'UNE OSMOSE INVERSE

(30) Priority: 30.08.1999 US 151497 P
(43) Date of publication of application: 12.06.2002
(73) Proprietor: Mossi & Ghisolfi International S.A., 1528 Luxembourg (LU)
(72) Inventor: DIAZ, Zaida, Houston, TX 77077 (US); RODDEN, John, B., Houston, TX 77059 (US)
(74) Representative: Gerbino, Angelo
(86) International application number: PCT/US2000/021674
(87) International publication number: WO 2001/016067

(56) References cited:
- US-A- 3 671 578
- US-A- 3 888 921
- DATABASE WPI Derwent Publications Ltd., London, GB; AN 1978-18376A XP002159814 & JP 53 007633 A (MITSUBISHI GOS CHEMICAL CO.; TOYO BOSEKI; MIZUSHIMA AROMA KK) 9 July 1976 (1976-07-09)

## Description

### Technical Field

This invention is related to a process for the production of purified 2,6-naphthalene dicarboxylic acid (hereafter referred to as 2,6-NDA) from a crude 2,6-NDA disproportionation product. More particularly, this invention is related to a novel method of separating and recycling byproducts in a process for producing 2,6-NDA from a disproportionation product that utilizes reverse osmosis and is industrially advantageous.

### Background Art

Aromatic carboxylic acids are highly useful organic compounds. They are useful as intermediates for the preparation of other organic compounds, and as monomers for the preparation of polymeric materials. In particular, the naphthalene carboxylic acids are used for preparing photographic chemicals and dyestuffs. Naphthalene dicarboxylic acids can also be used to prepare a variety of polyester and polyamide compositions. 2,6-NDA is a particularly useful aromatic carboxylic acid which can be reacted with ethylene glycol to prepare poly(ethylene-2,6-naphthalate). Polyesters prepared from 2,6-NDA have excellent heat resistance, gas barrier, and mechanical properties. Therefore, much research in the art has focused on methods of preparing 2,6-NDA. The production of 2,6-NDA from disproportionation product is described, for example, in U.S. 2,823,231 and U.S. 2,849,482.

Production of high purity 2,6-NDA from disproportionation product requires many process steps to separate impurities from the dipotassium salt of 2,6-NDA, hereafter referred to as 2,6-K2NDA, which is the 2,6-NDA precursor. The impurities include naphthalene, zinc oxide, and several naphthalene mono- and dicarboxylic acid salts. This complexity results in numerous byproduct streams that must be recycled to make the process less costly.

There have been different approaches to the separation of the dialkali metal salt products of disproportionation reactions and conversion of them into 2,6-NDA.

In U.S. 2,823,231, the method used to separate the dialkali metal salts of 2,6 -naphthalene dicarboxylic acid comprises dissolving the disproportionation conversion product mixture in water, filtering off insoluble impurities from the resulting solution, acidifying the filtrate with mineral or organic acid, such as sulfuric or hydrochloric acid, and separating the precipitated naphthalene-2,6-dicarboxylic acid from the acid solution. In U.S. 2,823,231 the dialkali metal salt of naphthalene 2, 6-dicarboxylic acid formed is converted into free naphthalene 2,6-dicarboxylic acid by acidification of said dialkali metal salt with a strong mineral acid.

U.S. 2,849,482 teaches acidifying an aqueous solution of the crude reaction product of the disproportionation or converting the crude alkali metal salt directly into the dichloride or into esters of naphthalene-2, 6 - naphthalene dicarboxylic acid in accordance with known methods.

In U.S. 3,631,096, to Phillips, salts formed by the reaction can be transformed into the corresponding free acids by acidifying the solution with organic or inorganic acids or by introducing carbon dioxide into the solution at atmospheric or elevated pressure, and then separating the free acids from the acidified solution. The individual reaction products may be separated from each other and isolated in pure form by methods that are based upon their different solubilities or volatilities and may thereafter, if desired, be transformed into their derivatives. The salt mixture produced by the reaction may also be transformed directly into derivatives of the acids, for example, into their esters or halides, and these derivatives may be purified, if desired, by fractional distillation.

U.S. 3,671,578, to Teijin, discloses that the monoalkali salt of 2,6-naphthalene dicarboxylic acid is easily disproportionated when heated in water or water-containing organic solvent, to form free dicarboxylic acid and by-product dialkali salt, and the former acid is precipitated.

In U.S. 3,952,052, to Phillips, there is disclosed a process for separating a disproportionation reaction product by forming a slurry comprising alkali metal salts of aromatic polycarboxylic acid and dispersant and a gaseous effluent, and then lowering the pressure, flashing the dispersant, and recovering said alkali metal salts of said polycarboxylic acids as solids from said separation zone.

U.S. 3,888,921, to Teijin Ltd., discloses a method for purifying a dialkali salt of crude 2,6-naphthalene dicarboxylic acid comprising precipitating 40 to 97 mol percent of the dialkali 2,6-naphthalene dicarboxylate dissolved in an aqueous solution substantially as a monoalkali salt of the 2,6-naphthalenedicarboxylic acid while maintaining the pH of said aqueous solution at a value not lower than 6.3, and separating the precipitate, and converting the separated precipitate to a 2,6-naphthalene dicarboxylic acid.

Canadian Patent 864587 discloses a process for the preparation of 2,6-NDA which comprises heating a monoalkali salt of 2,6-NDA in water or water-containing organic solvent causing disproportionation thereof into 2,6-NDA and a dialkali salt and separating the 2,6-NDA by a method that includes dissolving a rearrangement reaction product containing dialkali salt of 2,6 - naphthalene dicarboxylic acid in warm water, filtering off the insoluble matter therefrom, concentrating the remaining solution, whereby the filtrate is concentrated to such a degree that the precipitation yield of the dialkali salt precipitated when the concentrated liquid is cooled to room temperature reaches at least 70% and the purity of said precipitate exceeds 99%, passing gaseous carbon dioxide through the aqueous solution of the precipitate recovered from the concentrated liquid, and recovering the resulting precipitate,and the mother liquour containing the side product dialkali salt of 2,6-naphthalene dicarboxylic acid is recycled into the carbon dioxide reaction step.

U.S. 5,175,354 discloses a reaction step wherein 2,6-naphthalene dicarboxylic acid potassium salts are allowed to react with benzene-carboxylic acids in the presence of water to yield 2,6-NDA and benzene-carboxylic acid potassium salts and a separation step wherein the crystallized 2,6-NDA is separated from the benzene-carboxylic acid potassium salts dissolved in the aqueous solution and provides 2,6-NDA.

None of these references suggest the idea of incorporating reverse osmosis membranes into a process for purifying 2,6-NDA.

The Abstract of JP-A-53 007 633 discloses the use of reverse osmosis in a process for concentrating a solution of terephthalic acid.

There is a need in the art for alternative methods of separating the desired product and efficiently recycling byproducts. The purification process of the present invention provides an efficient way of separating and recycling byproducts which is advantageous.

### Disclosure of the Invention

In accordance with the foregoing the present invention comprises a process for purifying 2,6-naphthalene dicarboxylic acid produced by disproportionation and more efficiently recycling byproduct dipotassium salts which comprises:
a) Dissolving the disproportionation product of potassium naphthoate comprising the dipotassium salt of 2,6-NDA (K2NDA) in water, removing any residual disproportionation reaction medium, centrifuging the solution to remove the disproportionation catalyst, and removing acid salts other than 2,6-NDA by crystallization and/or carbon adsorption;
b) Contacting said aqueous solution of 2,6-K2NDA with carbon dioxide to form as a precipitate the monopotassium salt of 2,6-NDA (KHNDA) and an aqueous solution containing 2,3-KHNDA, K2NDA, and potassium bicarbonate;
c) Separating said monopotassium salt as a solid from said stream containing 2,3-KHNDA, K2NDA and potassium bicarbonate;
d) Disproportionating said monopotassium salt (KHNDA) to form solid 2,6-NDA and an aqueous solution containing K2NDA and potassium bicarbonate;
e) Separating said 2,6-NDA;
f) Concentrating said aqueous solution containing K2NDA and potassium bicarbonate from step (d) by reverse osmosis; and
e) Recycling concentrated K2NDA to step (b) and pure water to step (d).

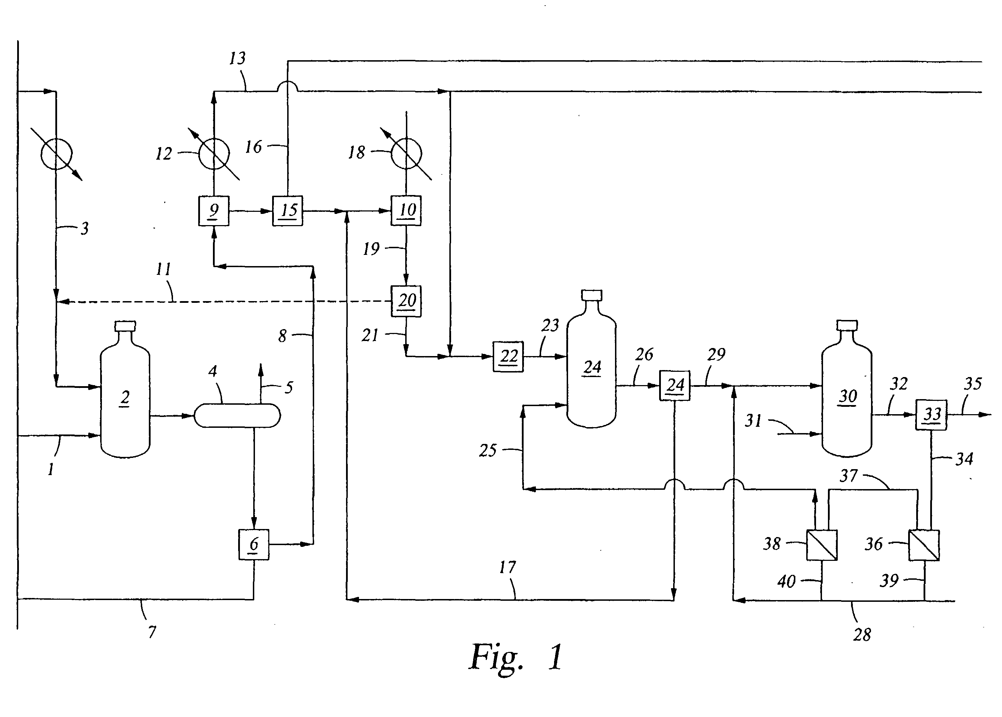

### Brief Description of the Drawing

The drawing is a process flow diagram illustrating the use of the process of the present invention as part of an integrated process for producing 2,6-naphthalene dicarboxylic acid.

### Detailed Description of the Invention

The novel process of the present invention for producing high purity 2,6-NDA begins with a disproportionation reaction product. This type reaction is described, for example, in U.S. 2,823,231 and U.S. 2,849,482.

The present invention is advantageously used in conjunction with a process for the production of 2,6-NDA by disproportionation of potassium naphthoate as described in copending U.S. Patent Application Ser. No. 60/151,577, incorporated by reference herein in the entirety. In that application the disproportionation effluent solids (in naphthalene) consist primarily of 2,6 K2NDA, 2,3 K2NDA (isomer intermediate), unreacted KNA, catalyst, and trace coke. After leaving the disproportionation reactor the solvent is flashed.

Next, the solid product comprising dipotassium salts of 2,6-NDA, K2NDA (2,6- and 2,3- isomers), unreacted KNA, catalyst, heavy by-products, any residual solvent, and trace coke enter a water wash. The organic salts are dissolved and the liquid is directed to a decanter and centrifuge to remove residual solvent, catalyst and coke particles. The ZnO catalyst is regenerated and recycled.

The next step in the process is crystallization of the dipotassium salt. The dipotassium salt of naphthalene dicarboxylic acid resulting from the disproportionation reaction contains at least 15% unconverted feed and intermediates. The liquid carrying the dipotassium salts of NDA, K2NDA (2,6- and 2,3- isomers), KHCO₃, and unreacted KNA, flows to a two-stage evaporative crystallization section, where the disalt of 2,6 NDA (2,6 K2NDA) is selectively precipitated.

The crystallization section rejects a mother liquor stream containing KHCO₃, unreacted KNA, and 2,3 K2NDA. Recovery of 2,6 K2NDA is approximately 90%, and the purity of the K2NDA leaving the second crystallizer is 99.9%+.

The purified K2NDA slurry is then redissolved with additional clean water and optionally treated with a solid adsorbing agent. Examples of solid adsorbing agents include activated carbon, alumina, magnesia or ion exchange resins. The use of activated carbon is especially preferred. The amount of the solid adsorbent to be used depends upon the amounts of impurities contained therein. A suitable amount of adsorbent would be in the range of 0.1 to 10 percent by weight, preferably 0.5 to 5 percent by weight, based on the K2NDA. By subjecting an aqueous solution of the dipotassium salt to a solid adsorbent, most residual trace impurities that could affect the color of the final product can be removed.

Next, the monopotassium salt of 2,6-NDA (KHNDA) is selectively precipitated from an aqueous solution of K2NDA (about 20%) by reacting said aqueous solution at 0 - 1,379 kPa (0-200 psi) CO₂ pressure, and 0-50°C for about 30 minutes. The reaction produces the solid mono-potassium salt of 2,6-NDA, 2,6-KHNDA, and also 2,3-KHNDA and potassium bicarbonate. 2,3-KHNDA is rejected from the 2,6-KHNDA crystals.

The CO₂ precipitation step effectively separates 2,6-KHNDA from 2,3-KHNDA, which remains in solution due to its higher. solubility. Examples 1-8 demonstrate this separation. The rejection of the 2,3-KHNDA is beneficial because, as a result, 2,3-KHNDA does not interfere with the separation of the 2,6-NDA from the K2NDA and the reverse osmosis of the present invention that takes place after the disproportionation of the 2,6-KHNDA.

Yields of 2,6-KHNDA better than 80% have been demonstrated at only 1 atm CO₂ pressure. The fact that the precipitation can be done effectively at modest pressure allows for centrifugation of the product without releasing pressure. The centrate also contains dissolved potassium bicarbonate and 2,3-KHNDA.

KHNDA solids are then diluted to 5-10% and disproportionated by reacting for less than an hour, preferably about 20 to 30 minutes at 150°C, under 344.75 kPa (50 psi) CO₂ pressure. The reactor effluent from this step is separated to give a 2,6-NDA solid, and a centrate containing predominantly 2,6-K2NDA and KHCO₃.

This centrate stream from the disproportionation of the monosalt, KHNDA, is the primary focus of the present invention. According to the present invention the K2NDA in the centrate stream would be very useful if recycled to the CO₂ precipitation step, however it has to be concentrated because the optimal salt concentration in the CO2 precipitation step is about 20 wt%, whereas it is less than 10 wt% in the KHNDA disproportionation step. Concentrating this solution by evaporating off water is very energy intensive and costly.

It has been discovered in the present invention that when the solid 2,6-NDA produced in the disproportionation of KHNDA is separated out, the remaining solution containing K2NDA and potassium bicarbonate can be concentrated via reverse osmosis and recycled to the CO₂ precipitation step very efficiently and economically. The reverse osmosis step produces a pure water stream that can be recycled to the disproportionation step, and a concentrated K2NDA solution that can be recycled to the CO₂ precipitation step. Any potassium present in forms such as potassium carbonate or potassium bicarbonate is also separated by the membrane for recycle.

The dipotassium salt should be concentrated to a wt% in the range of 10-30 wt% salt. In the examples of the present invention the target was 20 wt% salt.

The reverse osmosis membranes that are suitable for use in the process are those characterized by high flux and high salt rejection, hydrolytic stability, resistance to compaction under pressure, and resistance to chemical attack.

The membranes employed in the examples were thin film composite membranes. These membranes consist of three layers: a support web, a microporous polysulfone layer with controlled pore diameters, and an ultrathin polyamide coating which is the selective layer. The support web provides the major structural support; the interlayer provides a smooth surface for the selective layer. The selective layer is on the order of 0.2 microns and can withstand high pressures due to the support provided by the interlayer. Examples of suitable membranes are FT-30 and HP-31, commercially available from Rochem Environmental, Inc.

In the present invention it is necessary to increase pressure in conjunction with the use of the. membranes to achieve the desired concentration of the K2NDA. Suitable pressure is a pressure higher than the osmotic pressure of the solution. Good results were observed where a pressure in the range of 5,516 to 13,790 kPa g (800 to 2000 psig) was used. In some cases it is advantageous to use a pressure on the lower end of the range until most of the water is recovered, say 60-80%, and then employ a higher pressure. Examples 9-13 and 14-19 set forth data obtained for tests at low pressure and two-stage (low to high) pressure, respectively.

It has been found that the 2,6-NDA produced by this process is of high purity and contains only low levels of potassium. It has also been found'that potassium can be removed to even lower levels by washing the 2,6-NDA with water.

### Detailed Description of the Drawing

The drawing is a flow diagram showing one embodiment of the process of the present invention as part of a purification section for producing 2,6-NDA. It is understood the drawing is only intended as an illustration.

Referring to the Figure, solid product comprising dipotassium salts of NDA, K2NDA (2,6 and 2,3 isomers), unreacted KNA, catalyst, heavy by-products, and trace coke from which most of the reaction medium from the disproportionation reaction has been removed, represented by **1** enters water wash **2** where the organic salts are dissolved. Steam and 25% naphthalene can enter the water wash via **3** from another section of the process. The entire integrated process is discussed in detail in copending Ser. No. 60/151,577. The liquid is then directed to a decanter **4** to remove any residual solvent, catalyst and coke particles. Naphthalene and some solids exit the process at **5**, while an aqueous solution of crude K2NDA also containing solid ZnO catalyst is directed to a centrifuge **6.** ZnO catalyst exits the centrifuge through **7** and is recycled. The liquid carrying the mixed organic salts, including the crude K2NDA is directed through **8** to a two-stage evaporative crystallization section, **9** and **10**.

In the evaporative crystallization section 2,6-K2NDA is selectively precipitated from crude K2NDA product, rejecting KNA, 2,3-K2NDA, and KHCO₃. First, the crude K2NDA stream **8** and a recycle stream **11** containing KHCO₃ are added to evaporative crystallizer **9.** In evaporative crystallizer 9, 2,6-K2NDA is selectively precipitated as water is evaporated. The water vapor exits the crystallizer, and is condensed by overhead exchanger **12.** The water is then routed through line **13** to other portions of the finishing section in order to provide a dilution medium. The contents of the first evaporative crystallizer **9** exit through **14** to centrifuge **15.** In centrifuge **15,** mother liquor containing KNA, 2,3-K2NDA, and KHCO₃ are rejected, exit at **16,** and are recycled back to the salt formation reactor in another section of the integrated process. The K2NDA solids are combined with recycle stream **17** containing KHCO₃ and 2,6-K2NDA and added to the second stage evaporative crystallizer **10.** In **10** 2,6-K2NDA is again selectively precipitated as water evaporates and exits the crystallizer. The water is condensed by overhead exchanger **18** and is directed into line **13**. The purified K2NDA slurry leaves the second stage evaporative crystallizer through 19 and is directed to centrifuge **20.** In centrifuge **20** mother liquor containing KHCO₃ is separated from purified 2,6-K2NDA and recycled back to the first stage evaporative crystallizer **9** through **11.**

The purified solid 2,6-K2NDA is dissolved with water from overhead line **13** and transported through line **21** to an activated carbon guard bed **22.** The 2,6-K2NDA solution then passes through the activated carbon guard bed **22** to remove residual trace impurities that could affect the color of the final product.

The 2,6-K2NDA solution exits the activated carbon bed **22** via line **23** and is directed to the CO₂ precipitation reactor **24.** CO₂ is added to reactor **24** through line **25.** In reactor **24** the monopotassium salt of 2,6-NDA, KHNDA, is selectively precipitated from the 2,6-K2NDA solution. The KHNDA is then directed out of the reactor through line **26** to centrifuge **27.** The mother liquor, containing KHCO₃ and unreacted 2,6-K2NDA, is separated from the solid KHNDA and is recycled back to the second stage evaporative crystallizer **10** via line **17.** The solid KHNDA is slurried with water from recycle line **28** and directed through line **29** to disproportionation reactor **30.** CO₂ is added to reactor 30 through line 31. The KHNDA is reacted in the presence of 344.75 kPag (50 psig) CO₂ and about 150°C in disproportionation reactor **30** to form solid 2,6-NDA and 2,6-K2NDA. The reactor effluent from this step is directed through **32** to centrifuge **33**.

This is the point where the present invention provides a very efficient method of making the process more economical. The solid 2,6-NDA is separated from the mother liquor and exits through **35** to a section for further purification and reduction of potassium levels. The centrate containing predominantly 2,6-K2NDA is directed through **34** to a two-stage reverse osmosis section, **36** & **38**. In **36** the K2NDA feed enters a reverse osmosis stage operated at a lower pressure. Concentrate exits at **37** and is directed to a second reverse osmosis stage **38** operated at higher pressure, and permeate (water) exits at **39** and connects with a water recycle line which is directed back to the disproportionation step. The concentrate from **38** exits into line **25** which recycles back to the CO₂ precipitation step and water from the second stage reverse osmosis exits at **40.**

The present invention will be more clearly understood from the following examples. It is understood that these examples are presented only to illustrate some embodiments of the invention and are not intended to limit the scope thereof.

### EXAMPLES 1-8

Examples 1-8 were performed to investigate the separation of 2,3-KHNDA from 2,6-KHNDA in the CO₂ precipitation step. In these experiments, aqueous solutions containing 5% molar 2,3-K2NDA based on 2,6-K2NDA were contacted with CO₂ at 100°C and various CO₂ pressures. The results in Table 1 show that the precipitate obtained by this process contained essentially no 2,3-NDA impurity.

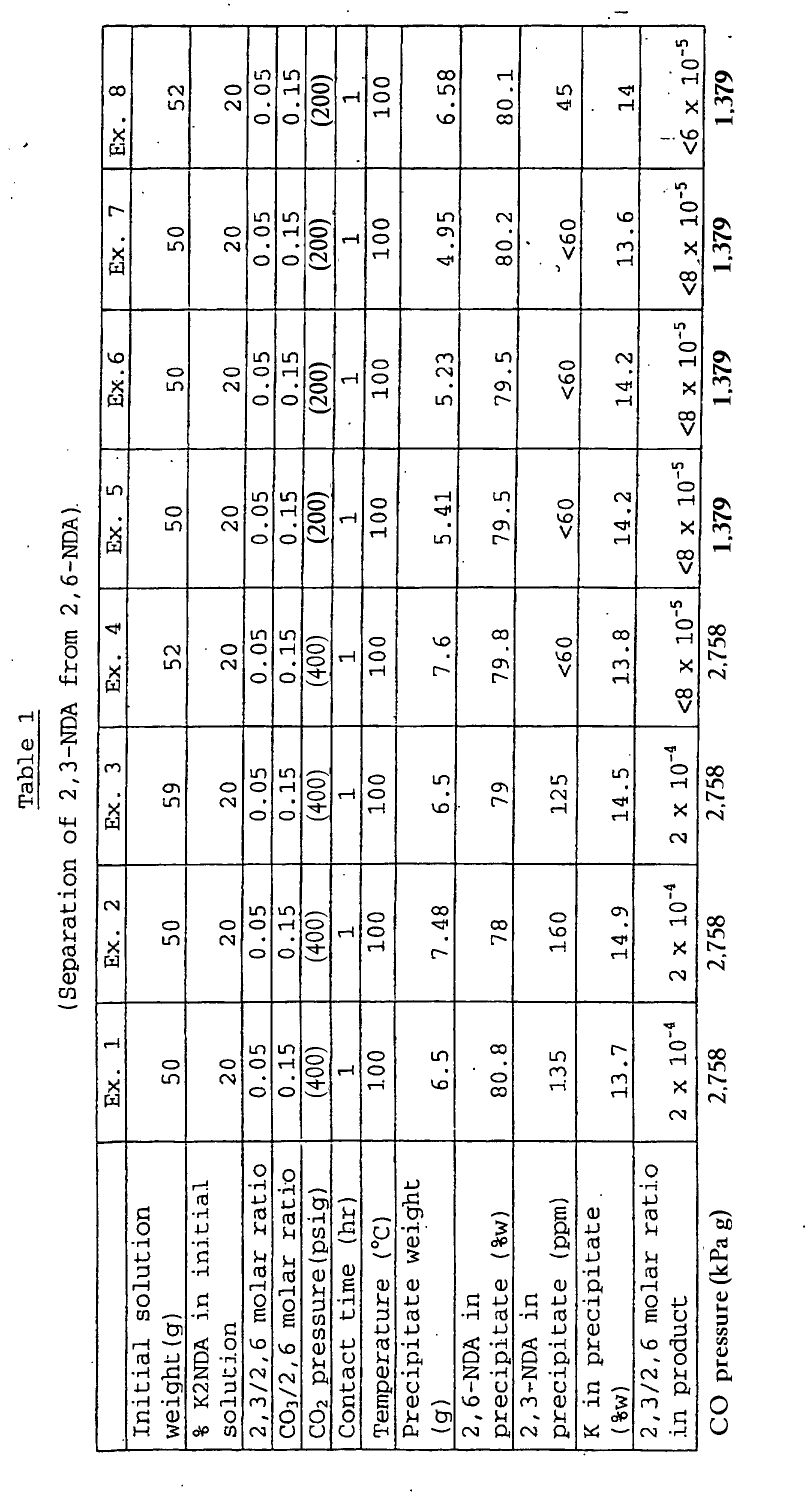

### EXAMPLES 9-13

Reverse osmosis experiments were carried out using a 3 wt% solution of 2,6-K2NDA. The pH and conductivity of the test solution were 9.2 and 16,100 µS/cm, respectively. A Rochem Disc Tube ™(DT) module, scaled down to 1/10^{th} of the standard 169 membrane module was used for all examples. Examples 9-13 were performed at low pressure using an FT30 membrane. In the low pressure test, the system was operated below 6,205 kPa g (900 psig). Examples 14-19 were carried out using an FT-30 membrane in a low pressure module and an HP31 membrane in a high pressure module. The low pressure module was operated below 6,205 kPa g (900 psig) up to a 75% water recovery, and then a switch was made to the high pressure module operated below 12,411 kPa g (1800 psig). The initial feed volume was 62 liters. Based on the calculated feed concentration of 3 wt%, a volume reduction requirement of 85% was assumed to achieve the goal of 20 wt% K2NDA in the resulting stream. The results obtained are set forth in Tables 2 and 3 and clearly show excellent salt rejection achieved. The flux rates obtained in these examples after normalization with respect to temperature and pressure range from 1019 to 2852 ℓ/m²-day (25 to 70 gal/sq.ft-day).

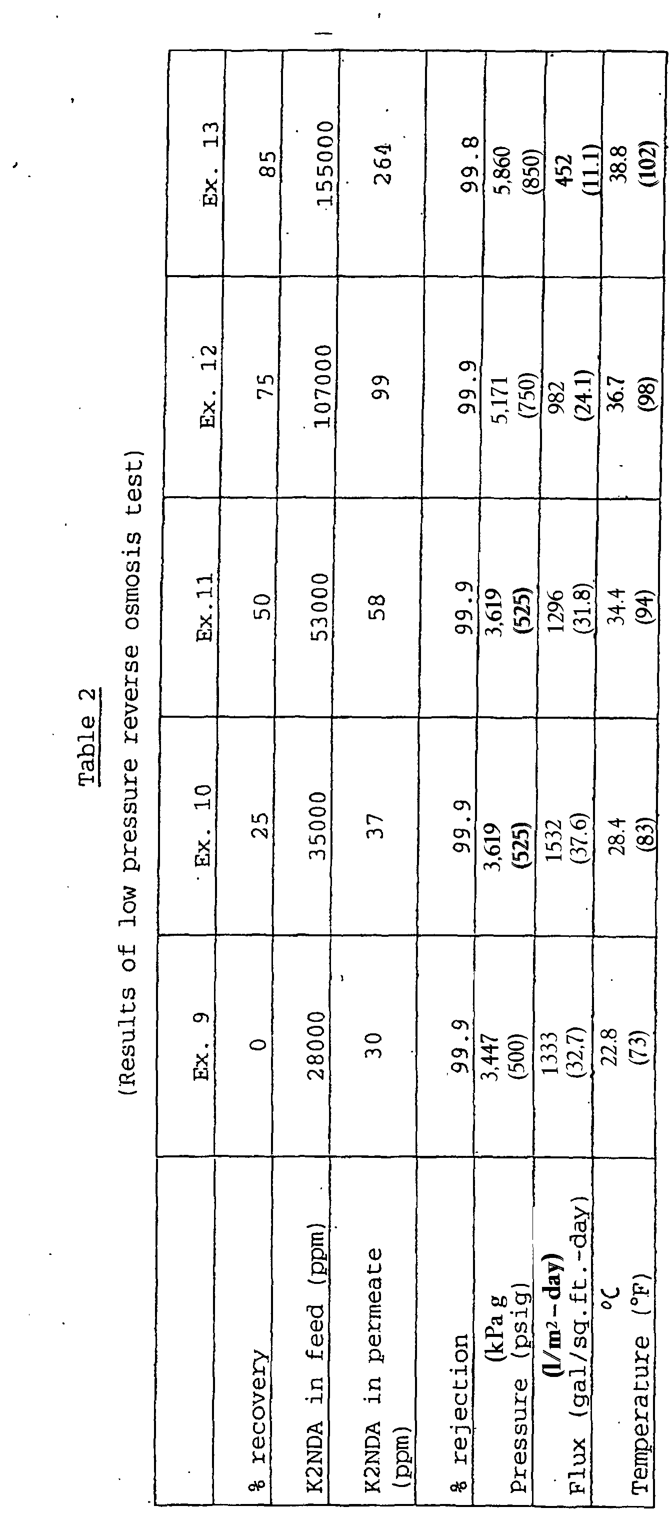

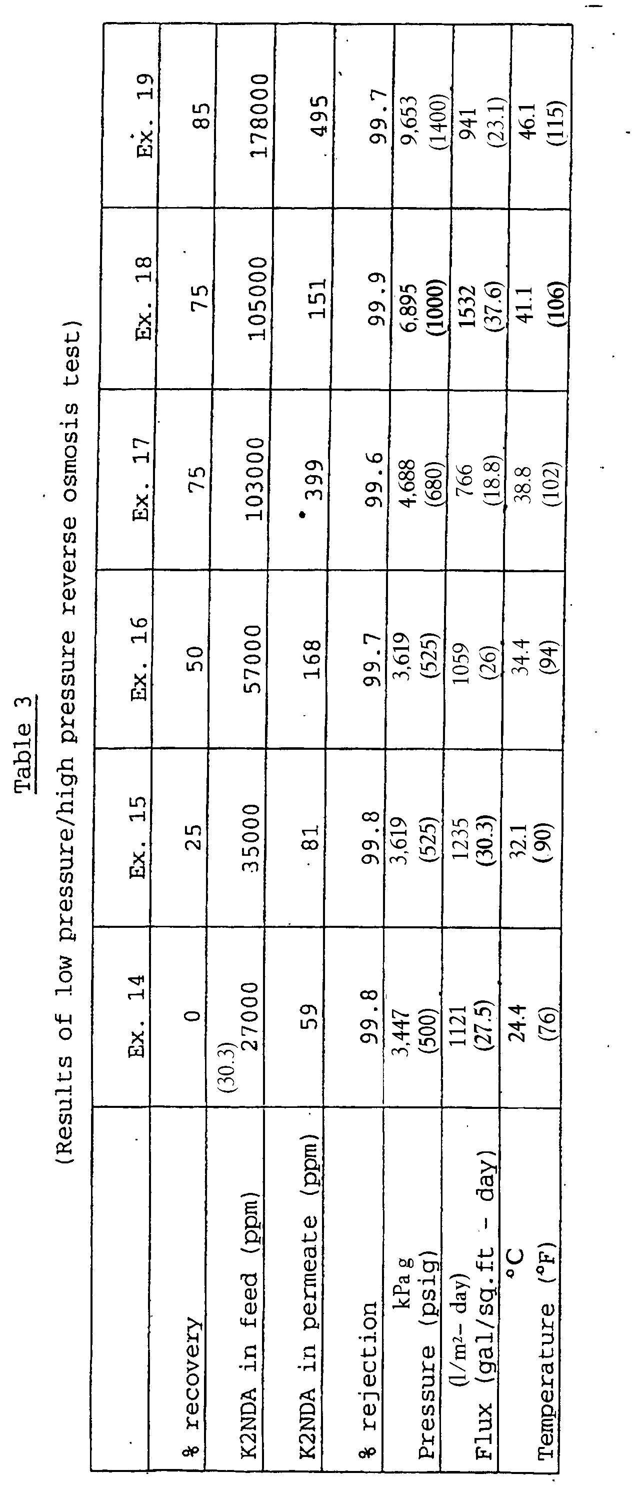

## Claims

1. A process for purifying 2,6-naphthalene dicarboxylic acid produced by disproportionation and more efficiently recycling byproduct dipotassium salts which comprises:
a) Dissolving a disproportionation product comprising the dipotassium salt of 2,6-NDA (K2NDA) in water, removing any remaining disproportionation reaction medium, centrifuging the solution to separate disproportionation catalyst, and removing acid salts other than 2,6-NDA by crystallization and/or carbon adsorption,
b) Contacting said aqueous solution of 2,6-K2NDA with carbon dioxide to form as a precipitate the monopotassium salt of 2,6-NDA (KHNDA) and an aqueous solution containing 2,3-KHNDA, K2NDA, and potassium bicarbonate;
c) Separating said monopotassium salt as a solid from said stream containing 2,3-KHNDA, K2NDA and potassium bicarbonate;
d) Disproportionating said monopotassium salt (KHNDA) to form 2,6-NDA and an aqueous solution containing K2NDA and potassium bicarbonate;
e) Separating said 2,6-NDA;
f) Concentrating said aqueous solution containing K2NDA and potassium bicarbonate from step (d) by reverse osmosis; and
g) Recycling concentrated K2NDA to step (b) and pure water to step (d).

2. The process of Claim 1 wherein the aqueous solution of step (f) is concentrated by reverse osmosis to a wt% of disalt of 10 -30 wt% dipotassium naphthalene dicarboxylic acid.

3. The process of Claim 2 wherein the aqueous solution of step (f) is concentrated by reverse osmosis to about 20 wt% dipotassium naphthalene dicarboxylic acid.

4. The process of Claim 1 wherein reverse osmosis of step (f) is accomplished using thin film composite membranes.

5. The process of Claim 4 wherein the membrane further comprises three layers consisting of a support web, a microporous polysulfone interlayer with controlled pore diameters, and an ultrathin polyamide coating selective layer.

6. The process of Claim 5 wherein the selective layer is on the order of 0.2 microns and can withstand high pressures due to the support provided by the interlayer.

7. The process of Claim 1 further comprising the reverse osmosis of step (f) being carried out under a pressure in the range of 3,447 to 13,790 kPa g (500 to 2000 psig).

8. The process of Claim 1 further comprising the reverse osmosis of step (f) being carried out in two stages.

9. The process of Claim 8 further comprising contacting the solution of dipotassium salts with a reverse osmosis membrane at a pressure of 3,447 to 6,205 kPa g (500 - 900 psig) for a period and then contacting the concentrate with a second reverse osmosis membrane at a pressure of 11,032 to 12,411 kPa g (1600 -1800 psig) for a period of time.

10. The process of Claim 9 further comprising contacting the solution of dipotassium salts with the first membrane a pressure below 6,205 kPag (900 psig) until about 70-80% of the water is recovered and then contacting the solution with the second membrane at a pressure below 12,411 kPa g (1800 psig).

## Patentansprüche

1. Verfahren zur Reinigung von 2,6-Naphtalen-Dikarbonsäure, produziert durch die Disproportionierung und das effizientere Recycling von Dikaliumsalzen als Nebenprodukt, umfassend:
a) Lösen eines Disproportionierungsprodukts, umfassend das Dikaliumsalz von 2,6-NDA (K2NDA), in Wasser, Entfernen restlicher Disproportionierungsreaktionsmedien, Zentrifugieren der Lösung zur Trennung des Disproportionierungskatalysators und Entfernen von sauren Salzen ausgenommen 2,6-NDA durch Kristallisation und/oder Kohlenstoffadsorption;
b) Kontaktieren der wässrigen Lösung von 2,6-K2NDA mit Kohlendioxid zur Bildung des Monokaliumsalzes von 2,6-NDA (KHNDA) als Niederschlag und einer wässrigen Lösung, die 2,3-KHNDA, K2NDA und Kaliumbikarbonat enthält;
c) Trennen des Monokaliumsalzes als Feststoff von dem Strom, der 2,3-KHNDA, K2NDA und Kaliumbicarbonat enthält;
d) Disproportionieren des Monokaliumsalzes (KHNDA) zur Bildung von 2,6-NDA und einer wässrigen Lösung, die K2NDA und Kaliumbicarbonat enthält;
e) Trennen des 2,6-NDA;
f) Konzentrieren der wässrigen Lösung, die aus Schritt (d) K2NDA und Kaliumbicarbonat enthält, durch Umkehrosmose; und
g) Rezyklieren von konzentrierter K2NDA zu Schritt (b) und reinem Wasser zu Schritt (d).

2. Verfahren nach Anspruch 1, wobei die wässrige Lösung des Schritts (f) durch Umkehrosmose zu einem Disalz-Gewichtsanteil von 10 - 30 Gewichtsprozent Dikaliumnapthalendicarbonsäure konzentriert wird.

3. Verfahren nach Anspruch 2, wobei die wässrige Lösung des Schritts (f) durch Umkehrosmose auf etwa 20 Gewichtsprozent Dikaliumnapthalendicarbonsäure konzentriert wird.

4. Verfahren nach Anspruch 1, wobei die Umkehrosmose des Schritts (f) durch Verwendung von Membranen aus Dünnverbundfolie ausgeführt wird.

5. Verfahren nach Anspruch 4, wobei die Membran des weiteren drei Schichten, bestehend aus einem Stützgewebe, einer mikroporösen Polysulphon-Zwischenschicht mit kontrollierten Porendurchmessern und einer selektiven ultradünnen Polyamid-Deckschicht umfasst.

6. Verfahren nach Anspruch 5, wobei die selektive Schicht in der Größenordnung von 0,2 Mikron ist und aufgrund der Unterstützung durch die Zwischenschicht hohen Drücken standzuhalten vermag.

7. Verfahren nach Anspruch 1, des weiteren umfassend die Umkehrosmose des Schritts (f), ausgeführt unter einem Druck in der Größenordnung von 3,447 bis 13,790 kPag (500 - 2000 psig).

8. Verfahren nach Anspruch 1, des weiteren umfassend die Umkehrosmose des Schritts (f), ausgeführt in zwei Phasen.

9. Verfahren nach Anspruch 8, des weiteren umfassend die Kontaktierung der Lösung von Dikaliumsalzen mit einer Umkehrosmosenmembran bei einem Druck von 3,447 bis 6,205 kPag (500 - 900 psig) während eines Zeitraums und dann Kontaktierung des Konzentrats mit einer zweiten Umkehrosmosemembran während eines Zeitraumes bei einem Druck von 11,032 bis 12,411 kPag (1600 - 1800 psig).

10. Verfahren nach Anspruch 9, des weiteren umfassend die Kontaktierung der Lösung von Dikaliumsalzen mit der ersten Membran bei einem Druck unter 6,205 kPag (900 psig), bis etwa 70 - 80% des Wassers gewonnen ist, und dann Kontaktierung der Lösung mit der zweiten Membran bei einem Druck unter 12,411 kPag (1800 psig).

## Revendications

1. Procédé de purification d'acide 2,6-naphtalène dicarboxylique produit par dismutation et recyclage plus efficace des produits secondaires sels de di-potassium, qui comprend :
a) la dissolution du produit de dismutation comprenant le sel de dipotassium de 2,6-NDA (K2NDA) dans de l'eau, le retrait de tout milieu résiduel de la réaction de dismutation, la centrifugation de la solution pour séparer le catalyseur de dismutation et le retrait des sels d'acides autres que le 2,6-NDA par cristallisation et/ou adsorption du carbone ;
b) la mise en contact de ladite solution aqueuse de 2,6-K2NDA avec du dioxyde de carbone pour former comme précipité, le sel de monopotassium de 2,6-NDA (KHNDA) et une solution aqueuse contenant le 2,'3-KHNDA, le K2NDA et du bicarbonate de potassium ;
c) la séparation dudit sel de monopotassium sous forme solide dudit flux contenant le 2,3-KHNDA, le K2NDA et le bicarbonate de potassium ;
d) le dismutation dudit sel de monopotassium (KHNDA) pour former le 2,6-NDA solide et une solution aqueuse contenant le K2NDA et du bicarbonate de potassium ;
e) la séparation dudit 2,6-NDA ;
f) la concentration de ladite solution aqueuse contenant le K2NDA et le bicarbonate de potassium à partir de l'étape (d) par osmose inverse ; et
g) le recyclage du K2NDA concentré de l'étape (b) et de l'eau pure de l'étape (d).

2. Procédé selon la revendication 1, dans lequel la solution aqueuse de l'étape (f) est concentrée par osmose inverse jusqu'à un pourcentage en poids de di-sel de 10 à 30 % en poids d'acide dipotassium naphtalène dicarboxylique.

3. Procédé selon la revendication 2, dans lequel la solution aqueuse de l'étape (f) est concentrée par osmose inverse jusqu'à environ 20 % en poids d'acide dipotassium naphtalène dicarboxylique.

4. Procédé selon la revendication 1, dans lequel l'osmose inverse de l'étape (f) est réalisée en utilisant des membranes composites à film fin.

5. Procédé selon la revendication 4, dans lequel la membrane comprend en outre trois couches constituées d'une trame de soutien, d'une couche intermédiaire microporeuse de polysulfone avec un diamètre de pores contrôlé, et une couche sélective à revêtement en polyamide ultrafin.

6. Procédé selon la revendication 5, dans lequel la couche sélective est de l'ordre de 0,2 microns et peut supporter des pressions élevées grâce au support fourni par la couche intermédiaire.

7. Procédé selon la revendication 1, comprenant en outre l'osmose inverse de l'étape (f) réalisée sous une pression de 3 447 à 13 790 kPa g (500 à 2000 psig).

8. Procédé selon la revendication 1, comprenant en outre l'osmose inverse de l'étape (f) réalisée en deux étapes.

9. Procédé selon la revendication 8, comprenant en outre la mise en contact de la solution de sels de dipotassium avec une membrane d'osmose inverse à une pression de 3 447 à 6 205 kPa g (500 à 900 psig) pendant une certaine durée, puis la mise en contact du concentré avec une deuxième membrane d'osmose inverse à une pression de 11 032 à 12 411 kPa g (1600 à 1800 psig) pendant une certaine durée.

10. Procédé selon la revendication 9, comprenant en outre la mise en contact de la solution de sels de dipotassium avec la première membrane à une pression inférieure à 6 250 kPa g (900 psig) jusqu'à ce qu'environ 70 à 80 % de l'eau soit récupérés, puis la mise en contact de la solution avec la deuxième membrane à une pression inférieure à 12 411 kPa g (1800 psig).
